# EUROPEAN PATENT SPECIFICATION

(11) **EP 2 830 656 B1**
(45) Date of publication and mention of the grant of the patent: **02.08.2017**
(21) Application number: 13711463.3
(22) Date of filing: 15.03.2013
(51) Int. Cl.: A61K 39/39

(54) **ADJUVANT**
ADJUVANS
ADJUVANT

(30) Priority: 26.03.2012 GB 201205237
(43) Date of publication of application: 04.02.2015
(73) Proprietor: The University Court Of The University of Edinburgh, Edinburgh EH8 9YL (GB)
(72) Inventor: HOWIE, Sara, Edinburgh EH16 4JT (GB); DONALDSON, Kenneth, Edinburgh EH16 4JT (GB); CHO, Wan Seob, Edinburgh EH16 4JT (GB)
(74) Representative: Gibbs, Richard
(86) International application number: PCT/GB2013/050664
(87) International publication number: WO 2013/144562

(56) References cited:
- US-A1- 2011 123 620
- US-A1- 2011 123 632
- PUSIC KAE ET AL: "Blood stage merozoite surface protein conjugated to nanoparticles induce potent parasite inhibitory antibodies", VACCINE, vol. 29, no. 48, November 2011 (2011-11), pages 8898-8908, XP002698053,
- CHO W-S ET AL: "NiO and Co3O4 nanoparticles induce lung DTH-like responses and alveolar lipoproteinosis", EUROPEAN RESPIRATORY JOURNAL, MUNKSGAARD INTERNATIONAL PUBLISHERS, COPENHAGEN, DK, vol. 39, no. 3, 1 March 2012 (2012-03-01), pages 546-557, XP008162472, ISSN: 0903-1936, DOI: 10.1183/09031936.00047111 cited in the application
- CHO WAN-SEOB ET AL: "Adjuvanticity and toxicity of cobalt oxide nanoparticles as an alternative vaccine adjuvant", NANOMEDICINE, FUTURE MEDICINE LTD., LONDON, GB, vol. 7, no. 10, 1 October 2012 (2012-10-01), pages 1495-1505, XP008162594, ISSN: 1743-5889, DOI: 10.2217/NNM.12.35 [retrieved on 2012-07-20]

## Description

### FIELD OF THE INVENTION

The present invention provides novel adjuvants for use in altering, modulating or augmenting immune responses in human or animal subjects. The invention further provides compositions and vaccines comprising the same.

### BACKGROUND OF THE INVENTION

Vaccines are considered to be the ultimate immunological tool in the prevention of infectious disease by providing a long-lasting protective immunity with a good antibody response [1;2]. The role of the adjuvant is to enhance the specific response of the immune system to a minimal amount of the antigen supplied through activation of antigen presenting cells [3]. The ideal adjuvant would not be in itself antigenic and would boost immune responses whilst not inducing overreaction of the immune system which could harm the patient (such as inducing excess local inflammation, allergy or delayed type hypersensitivity).

Due to safety concerns, licensed adjuvants are very limited and pain and inflammation at sites of injection are common side effects of the use of adjuvants in clinical vaccines. The adjuvants most widely used in human vaccines are aluminium containing [4] such as alum, a mineral salt, usually made up as Al(OH)₃ or Al(PO)₄. Alum adjuvants are thus metal oxide particulates.

In mice, aluminium containing adjuvants induce humoral immunity predominantly by promoting Th2 type responses whilst having a poorer ability to simulate Th1 dependent antibody. Adjuvants of this type do not usually induce strong cell-mediated immune responses particularly cytotoxic T-cell responses[4] although they have been reported to prime cytotoxic CD8 responses in the presence of additional lipid adjuvant [5]. Therefore, alum containing adjuvant is suitable for responses against protein antigens or inactivated organisms but is not optimal for inducing responses to intracellular pathogens [1-4].

Nanoparticles (NP) have been expanded to a wide range of biomedical applications owing to their unique physicochemical properties compared to bulk chemicals [6]. For example, NP have greater surface area and high protein binding affinity by electrostatic interaction [7]. NP also can penetrate deep into tissue and enhance cellular uptake as well as escape lysosomal compartments [7-9]. These unique properties have lead to NP being considered as potential adjuvants [10;11]. The size of NP appears to affect adjuvant ability and smaller NP have been shown to produce greater antibody and cellular immune responses [12]. However most of the NP candidates for vaccine adjuvants are organic substances [13].

In murine asthma models, multi-walled carbon nanotubes [14], diesel exhaust particles [15], and nano-sized ultrafine carbon black [16] were reported to increase serum IgE levels by an adjuvant-like mechanism. In comparison with data reported using alum containing adjuvants [17;18], nano-sized carbon black showed a similar adjuvant effect with IgG1 and IgE increases [16].

It has been shown that nickel oxide NP (NiONP) and cobalt oxide NP (Co₃O₄NP) are capable of inducing Th1 associated cytokine responses in the lungs of female Wistar rats [19].

Pusic Kae et al., (2011: Vaccine; vol. 29, no. 48, p8898-8908) describes incorganis CdSe/ZnS nanoparticles for use as a vaccine delivery system. US2011/123620 discloses vaccine nanoparticles of silicon dioxide for the immunotherapy of cancer. The present invention seeks to obviate one or more of the problems associated with the prior art and to provide additional adjuvants suitable for use with vaccines against diseases in which cell-mediated immune responses are important for prevention or cure.

### SUMMARY OF THE INVENTION

The present invention is based on the identification of the adjuvant properties of inorganic (metal/metal oxide) nanoparticles. The subject-matter of the invention is defined by the claims. In a first aspect, the present invention provides an adjuvant comprising cobalt nanoparticles and/or cobalt oxide nanoparticles.

In one embodiment, the adjuvant may not comprise alum.

Advantageously, the adjuvants of this invention may comprise one or more types of metal nanoparticle. For example, the adjuvants described herein may comprise cocktails or mixtures of different types of inorganic (metal oxide) nanoparticle. In one embodiment, the adjuvants described herein may further comprise a quantity of an alum based adjuvant.

As such, an embodiment of this invention provides an adjuvant comprising, consisting or consisting essentially of cobalt nanoparticles and/or cobalt oxide nanoparticles.

The adjuvant of this invention may comprise, consist or consist essentially of cobalt nanopaticles. In one embodiment, the cobalt nanoparticles comprise cobalt oxide. Cobalt oxide nanoparticles suitable for use as adjuvants of this invention may comprise, for example, cobalt (II,III) oxide (Co₃O₄) nanoparticles.

In view of the above, this invention provides an adjuvant comprising, consisting or consisting essentially of cobalt and/or cobalt (II,III) oxide (Co₃O₄)) nanoparticles.

One of skill will appreciate that other forms of cobalt oxide exist and these may find similar application as adjuvants - accordingly, the adjuvants described herein may further comprise or may consist or consist essentially of cobaltous oxide (Cobalt (II) oxide: CoO) and/or cobaltic oxide (Cobalt (III) oxide: Co₂O₃).

The inventors have discovered that adjuvants comprising inorganic nanoparticles, particularly those comprising metal nanoparticles such as cobalt nanoparticles, induce a balanced Th1 and Th2 type response when administered *in vivo.* This is in contrast to other adjuvants, including, for example, those comprising alum, which induce only a Th1 or a Th2 response. One of skill will appreciate that while Th1 responses are effective in assisting clearance of intracellular pathogens, most adjuvants elicit Th2 responses which are more effective in the clearance of extracellular pathogens. As such, the inorganic or cobalt nanoparticle adjuvants of this invention can be used with a wide variety of vaccines and are particularly suited for use with vaccines against diseases in which humoral and/or cell-mediated immune responses are important for prevention or cure.

In addition, the inventors have observed that in contrast to alum based adjuvants (which largely comprise or consist of microparticulate alum - i.e. alum particles of between 0.1µm and 100µm), the inorganic (cobalt based) nanoparticle adjuvants of this invention induce immune responses which comprise much less of the 'allergic' IgE immunoglobulin isotype. This is important as it reduces hypersensitivity reactions following adjuvant administration.

As a further advantage, the inventors have discovered that inorganic nanoparticle based adjuvants - such as, for example, the cobalt nanoparticle adjuvants described herein, induce less inflammation at both the sensitisation and challenge sites. This has the advantage of reducing the painful swelling which can often follow the administration of an adjuvant such as alum.

In view of the above, and without wishing to be bound by theory, as a consequence of the induction of balanced TH1 and TH2 immune responses, reduced toxicity as compared to alum based adjuvants and the relative lack of allergic side effects, inorganic nanoparticles (for example metal nanoparticles comprising cobalt and/or cobalt oxide (specifically Co₃O₄)), represent versatile adjuvants suitable for use with vaccines where either a Th1 or a Th2 response or both Th1 and Th2 responses are needed to clear pathogens.

The term nanoparticle encompasses particles which are between about 1nm and about 100nm in diameter. For example, the nanoparticles may be about (or have an average diameter of) 1 nm, 5 nm, 10 nm, 15 nm, 20 nm, 25 nm, 30 nm, 40 nm, 50 nm, 60 nm, 70 nm, 80 nm, 90 nm , 95nm or 99nm. In one embodiment, the nanoparticles of an adjuvant composition provided by this invention may have an average diameter of about 15 nm, 16 nm, 17 nm, 18 nm, 19 nm or 20 nm. Where the nanoparticle comprise cobalt, they may have a diameter (or an average diameter) of about 17 nm, 17.5 nm, 18 nm, 18.1 nm, 18.2 nm, 18.3 nm, 18.4 nm, 18.5 nm, 18.6 nm, 18.7 nm, 18.8 nm, 18.9 nm, 19 nm, 19.5 nm or 20 nm.

In one embodiment, cobalt nanoparticles of the adjuvants described herein (including cobalt (II,III) oxide nanoparticles) have an average diameter of about 18.4 ± 5.0 nm,.

Also described herein is the use of the inorganic metal nanoparticles (including the cobalt/cobalt oxide) nanoparticles described herein as adjuvants.

In a second aspect, the invention provides inorganic cobalt nanoparticles and/or cobalt oxide nanoparticles for use as adjuvants. By way of example, the invention may provide Co₃O₄ nanoparticles and Co₃O₄ nanoparticles for use as adjuvants.

In a third aspect, the adjuvants provided by this invention may be provided as compositions. In one embodiment, an adjuvant composition of this invention may comprise one or more components selected from the group consisting of:
(i) cobalt nanoparticles and/or cobalt oxide nanoparticles;
(ii) additional adjuvant components (for example an alum based adjuvant); and
(iii) a pharmaceutically or physiologically acceptable excipient, solvent, carrier or diluent.

Suitable excipients may include, for example oil and one of skill will appreciate that the term "oil" may include alkanes, alkenes, alkynes, and their corresponding acids and alcohols, the ethers and esters thereof, and mixtures thereof. The individual compounds of the oil are light hydrocarbon compounds, i.e., such components have 6 to 30 carbon atoms. The oil can be synthetically prepared or purified from petroleum products. The "oil" may have a straight or branched chain structure. It may be fully saturated or have one or more double or triple bonds. Some non-metabolizable oils for use in the present invention include mineral oil, paraffin oil, and cycloparaffins, for example.

The term oil is also intended to include "light mineral oil," i.e., oil which is similarly obtained by distillation of petrolatum, but which has a slightly lower specific gravity than white mineral oil. Other "oil" for use as excipients may include metabolizable (non-toxic) oils. Oils of this type may include, for example vegetable oils, fish oils, animal oils or synthetically prepared oils which can be metabolized by the body of the subject (human or animal) to which the adjuvant will be administered and which are non-toxic. Sources for vegetable oils include nuts, seeds and grains.

One of skill will appreciate that any of the oil excipients described herein may be provided as oil-in-water, water-in-oil or water-in-oil-in-water emulsion forms.

An oil-in-water emulsion provided by the present invention is composed of an AMPHIGEN(R) formulation. This formulation comprises an aqueous component, lecithin, mineral oil, and surfactants. Patents describing the components of the formulation include US 5,084,269 and US 6,572,861.

Typically, the oil component of the present invention is present in an amount from 1 % to 50% by volume; or in an amount of 10% to 45%; or in an amount from 20% to 40%.

Other components of the adjuvant compositions described herein can include pharmaceutically acceptable excipients, such as carriers, solvents, and diluents, isotonic agents, buffering agents, stabilizers, preservatives, vaso-constrictive agents, antibacterial agents, antifungal agents, and the like. Typical carriers, solvents, and diluents include water, saline, dextrose, ethanol, glycerol, oil, and the like. Representative isotonic agents include sodium chloride, dextrose, mannitol, sorbitol, lactose, and the like. Useful stabilizers include gelatin, albumin, and the like.

Surfactants are used to assist in the stabilization of the emulsion selected to act as the carrier for the adjuvant and antigen. Surfactants suitable for use in the present inventions include natural biologically compatible surfactants and non- natural synthetic surfactants. Biologically compatible surfactants include phospholipid compounds or a mixture of phospholipids. Preferred phospholipids are phosphatidylcholines (lecithin), such as soy or egg lecithin. Lecithin can be obtained as a mixture of phosphatides and triglycerides by water-washing crude vegetable oils, and separating and drying the resulting hydrated gums. A refined product can be obtained by fractionating the mixture for acetone insoluble phospholipids and glycolipids remaining after removal of the triglycerides and vegetable oil by acetone washing. Alternatively, lecithin can be obtained from various commercial sources. Other suitable phospholipids include phosphatidylglycerol, phosphatidylinositol, phosphatidylsehne, phosphatidic acid, cardiolipin, and phosphatidylethanolamine. The phospholipids may be isolated from natural sources or conventionally synthesized.

Non-natural, synthetic surfactants suitable for use in the present invention include sorbitan-based non-ionic surfactants, e.g. fatty-acid-substituted sorbitan surfactants (commercially available under the name SPAN(R) or ARLACEL(R)), fatty acid esters of polyethoxylated sorbitol (TWEEN(R)), polyethylene glycol esters of fatty acids from sources such as castor oil (EMULFOR(R)); polyethoxylated fatty acid (e.g., stearic acid available under the name SIMULSOL M-53(R)), polyethoxylated isooctylphenol/formaldehyde polymer (TYLOXAPOL(R)), polyoxyethylene fatty alcohol ethers (BRIJ(R)); polyoxyethylene nonphenyl ethers (TRITON(R) N), polyoxyethylene isooctyl phenyl ethers (TRITON(R) X).

Generally speaking, the surfactant, or the combination of surfactants, if two or more surfactants are used, is present in the emulsion in an amount of 0.01 % to 10% by volume, preferably, 0.1 % to 6.0%, more preferably 0.2% to 5.0%.

As used herein, "a pharmaceutically-acceptable carrier" includes any and all solvents, dispersion media, coatings, adjuvants, stabilizing agents, diluents, preservatives, antibacterial and antifungal agents, isotonic agents, adsorption delaying agents, and the like. The carrier(s) should be "acceptable" in the sense of being compatible with the other components of the compositions and not deleterious to the subject. Typically, the carriers will be will be sterile and pyrogen-free, and selected based on the mode of administration to be used. It is well known by those skilled in the art that certain formulations of pharmaceutically acceptable carrier comprise those carriers approved in the applicable regulations promulgated by the United States (US) Department of Agriculture or US Food and Drug Administration, or equivalent government agency in a non-US country. Therefore, the pharmaceutically accepted carrier for commercial production of the compositions may be a carrier that is already approved or will be approved by the appropriate government agency in the US or foreign country.

The compositions may optionally include compatible pharmaceutically acceptable (i.e., sterile or non-toxic) liquid, semisolid, or solid diluents that serve as pharmaceutical vehicles, excipients, or media. Diluents can include water, saline, dextrose, ethanol, glycerol, and the like. Isotonic agents can include sodium chloride, dextrose, mannitol, sorbitol, and lactose, among others. Stabilizers include albumin, among others.

The compositions can also contain antibiotics or other preservatives, including, for example, gentamicin, merthiolate, or chlorocresol. The various classes of antibiotics or preservatives from which to select are well known to the skilled artisan.

In one embodiment, the adjuvant compositions of this invention may comprise one or more additional adjuvant components. For example, an adjuvant composition may, in addition to the inorganic (cobalt) nanoparticles described herein, comprise one or more adjuvants selected from the group consisting of alum (comprising aluminium hydroxide/phosphate); oil-based adjuvants and organic adjuvants (for example squalene).

In one embodiment, the adjuvant and/or adjuvant compositions described herein may be provided in lyophilised form or as lyophilised compositions. One of skill will appreciate that lyophilised adjuvant/adjuvant compositions of this invention may be reconstituted using any of the pharmaceutically acceptable carriers/diluents and/or excipients described herein.

Advantageously, the adjuvants and adjuvant compositions described herein may further comprise one or more preservative agents - including, for example, a cryopreservative agent.

Adjuvants are frequently used as components of vaccine compositions - particularly where the antigen of the vaccine is poorly immunogenic. One of skill will appreciate that adjuvants may be used to alter, modulate, modify or augment the effects of a vaccine so as to stimulate a suitable or appropriate immune response - one which assists in the clearance of a pathogen from a human or animal host. As such, in a further aspect, this invention extends to vaccines for human or animal use, wherein said vaccines comprise or are formulated with, an adjuvant described herein.

In a further embodiment, this invention provides the adjuvants of this invention (namely, cobalt based adjuvants) for use in enhancing immune responses in human or animal subjects. In one embodiment, the adjuvants of this invention are administered together or concurrently with, a vaccine. Typically, the adjuvants are formulated together with a vaccine for administration to a human or animal subject. In other embodiments, the adjuvants for use may be formulated as separate compositions (together with suitable pharmaceutical carriers, diluents and/or excipients) to be administered to human or animal subjects before or after administration of a vaccine.

One of skill will appreciate that the references to "vaccine" used herein may encompass any antigen preparation for use in raising immune responses in human or animal hosts. Many different types of vaccine are currently in use and many of these are formulated/combined or administered (concurrently or separately) with adjuvants to improve, augment or modify the nature of the raised immune response. In some cases, an adjuvant is exploited as a means to improve an immune response in a human or animal host. For example an adjuvant may be used to improve the immune response raised by low (sub-optimal) doses of antigen. In this regard, an adjuvant/adjuvant composition of this invention may be combined with sub-optimal doses of a vaccine or antigen - the vaccine or antigen serving to increase the immune response raised by the sub-optimal dose. When administered together or with an adjuvant or adjuvant composition of this invention, the immune response raised by a sub-optimal vaccine/antigen dose, may be comparable to an immune response raised by administration of an optimal dose of vaccine/antigen alone (i.e. without adjuvant).

In one embodiment, viral and/or bacterial antigen vaccines may be combined or administered (concurrently or separately) with the adjuvants/adjuvant compositions described herein. For example vaccines against, for example, forms of 'flu', diphtheria, Whooping cough, measles, mumps, rubella, polio, tuberculosis, meningitis, tetanus, yellow fever, rabies may be combined or administered with the adjuvants described herein.

Also described herein are (i) methods of raising immune responses and/or (ii) methods of enhancing, modulating or augmenting immune responses in human or animal subjects, said methods comprising the step of administering a vaccine and an adjuvant provided by the first aspect of this invention, to a human or animal subject in need thereof.

The adjuvant may be formulated with a vaccine such that it is administered to a human or animal host therewith. Alternatively, the adjuvant may be formulated as a separate composition such that it can be administered separately from the vaccine or concurrently therewith.

### DETAILED DESCRIPTION

The present invention will now be described in detail with reference to the following figures which show:
Figure 1: The shape and size of Co₃O₄NP (A) and I-alum (B). Particles were dispersed in distilled water and measured by a transmission electron microscopy.
Figure 2: Cytotoxicity of NPs or I-alum to RAW264.7 cells. Particles were added (concentrations given were in µg/ml) to RAW264.7 cells for 24 h with or without 1% OVA. Levels of lactate dehydrogenase (LDH) were measured for cytotoxicity. Note that I-Alum showed around 6 times more cytotoxicity compared to Co₃O₄NP and OVA increased the toxicity of particles. Data are mean ± SEM (*n* = 4 per group). The cytotoxicity data of particles treated with 1% OVA or without OVA were compared with their respective controls. ^{***}*P* < 0.001.
Figure 3: OVA-specific IgG1 levels in serum 7 days after second subcutaneous sensitisation. Other immunoglobulins (IgG2a, IgE, IgA, and IgM) showed no increases (data not shown). Data are mean ± SEM (*n* = 5 per group).
Figure 4: OVA-specific immunoglobulin levels present in serum 7 days after intraperitoneal challenge with ovalbumin. Immunoglobulins tested for were (A) IgG1, (B) IgG2a, (C) IgE, (D) IgA, and (E) IgM. Data are mean ± SEM (representative data, *n* = 5 per group). (F) Proliferation of spleen cells 7 days after intraperitoneal challenge with OVA. ³H-thymidine incorporation of cells cultured with medium only or in the presence of 10µM OVA.
Figure 5: OVA-specific IgG1 levels in peritoneal lavage fluid 7 days after challenge with ovalbumin. Other immunoglobulins (IgG2a, IgE, IgA, and IgM) showed no increases (data not shown). Data are mean ± SEM (*n* = 5 per group).
Figure 6: Levels of cytokine in the peritoneal lavage fluid collected 7 days after challenge. (A) IFN-γ; (B) IL-1β. Data are mean ± SEM (*n* = 5 per group). ^{*}*P <* 0.05 compared to PBS treatment group.
Figure 7: Differential cell counts of the peritoneal lavage fluid collected 7 days after challenge. Data are mean ± SEM (*n* = 5 per group). ^{*}*P* < 0.05, *^{**}P* < 0.01, and *^{***}P* < 0.001 compared to PBS treatment group.
Figure 8: The representative histology of injection site 7 days after challenge. A, PBS; B, NP-OVA; C, I-alum-OVA; D, NP alone. Note that NP-OVA and NP alone group showed mild inflammation (arrow) along with the deposition of the NPs (black spots) whilst I-alum-OVA showed massive inflammation and necrosis (arrow).
Figure 9: Graph showing the number of dendritic cells expressing certain markers.
Figure 10: Graph showing the cytotoxic response to cobalt oxide nanoparticles
Figure 11: Graph showing the cytotoxic response to Imject Alum
Figure 12: Graph showing cytotoxicity of DC in response to Cobalt Oxide NP ±1 % OVA.
Figure 13: Graph showing cytotoxicity of DC in response to Imject Alum ±1% OVA.
Figure 14: Graph showing IL-1β generation by DC treated with Cobalt Oxide NP ±1% OVA.
Figure 15: Graph showing IL-1β generation by DC treated with Imject Alum ±1% OVA.

### Example 1

As so very few adjuvants are capable of eliciting a Th1 response it was considered that NPs could be utilized to improve Th1 immunity against a model antigen, ovalbumin (OVA).

We selected Co₃O₄NP as a candidate adjuvant because Co₃O₄NP produced a Th1 response without the severe delayed-type hypersensitivity pathology seen with NiONP[19]. The aim was to determine whether Co₃O₄NP could be suitable for use as an adjuvant by inducing a balanced Th1 and Th2 response to the model antigen OVA given subcutaneously to female C57BL/6 mice.

To this end we compared anti-OVA responses induced by Co₃O₄NP to those induced by a commercially available aluminium containing adjuvant. The adjuvant chosen was Imject alum (I-alum), a metal particulate adjuvant widely used for murine experiments, which contains a 50/50 mixture of aluminium hydroxide and magnesium hydroxide and inactive stabilisers. In addition to ovalbumin-specific responses, toxicity was also assessed at the injection site and cytotoxicity for antigen presenting cells was evaluated using a mouse macrophage cell line.

### MATERIALS & METHODS

All chemicals and reagents were purchased from Sigma-Aldrich (Poole, UK) unless otherwise stated.

### Characterization and dispersion of particles.

The size of Co₃O₄NP (Nanostructural and Amorphous Materials, TX, USA) and Imject Alum (Thermo Scientific, Cramlington, UK) was measured using a transmission electron microscopy (JEM-1200EX II, JEOL, Tokyo, Japan). The hydrodynamic sizes of particles were measured using a particle size analyser (90Plus/BI-MAS; Brookhaven Instruments Corporation, New York, USA) in the presence of various concentrations of a dispersion medium. For *in vivo* study, hen egg ovalbumin (grade V) was used as a dispersion medium for Co₃O₄NP + OVA (NP-OVA) and I-alum + OVA (I-alum-OVA) whilst heat-inactivated serum (final concentration: 5%) collected from healthy C57BL/6 mice was used for NP-alone. The levels of endotoxin in the particle suspensions were measured using a Limulus Amebocyte Lysate assay (Cambrex, MD, USA). The solubility of Co₃O₄NP and I-alum was tested in the acidic artificial lysosomal fluid (pH 5.5) or basic artificial interstitial fluid (pH 7.4) according the previously described method[19].

### Rationale for dose selection.

Based on our previous study, we selected the dose of I-alum as 2.0 mg per mouse[17]. In contrast, the dose of Co₃O₄NP was selected as 25 µg per mouse based on the inflammogenicity in the lung; instillation of 419 µg/rat of Co₃O₄NP showed acute neutrophilic inflammation and chronic lymphocytic/neutrophilic inflammation with alveolar lipoproteinosis[19].

### Cytotoxicity assay (LDH).

Mouse macrophage cell line, RAW264.7 was cultured at 37°C with 5% CO₂ in Dulbecco's Modified Eagle's Medium (DMEM) with L-Glutamine, antibiotics (penicillin and streptomycin) and 10% heat-inactivated foetal bovine serum (FBS). Cells were detached using an accutase and seeded at 5 × 10⁵ in 12-well plates (Corning, NY, USA) for experiments. The cytotoxic effects of NPs and alum were measured using a lactate dehydrogenase (LDH) assay kit (Roche Applied Science, Sussex, UK) according to the manual instruction. As benchmark particles, TiO₂NP (30.5 ± 1.8 nm) (Nanostructural and Amorphous Materials, TX, USA) were used. NP or I-alum solutions with or without OVA (1%) were treated to RAW264.7 cells for 24 h. Each NP was tested at concentrations of 10, 30, 100 and 500 µg/ml whilst I-alum was tested at concentrations of 80, 240, and 800 µg/ml. After incubation, cell-free culture supernatants were centrifuged at 17000 × g for 20 min to get rid of particles. The cytotoxicity was calculated as percentage compared to the positive control (0.1% Triton-X).

### Animal immunisation.

Six-week old female C57BL/6 mice were obtained from Harlan Laboratories (Hillcrest, UK). All animals were maintained and handled under a specific license granted by the UK Home Office to one of the authors (SEMH) that ensures humane treatment and alleviation of suffering in all animal experiments. After 1 week to acclimatise, mice (5 mice per group) were immunised twice at 2 weeks interval. Each mouse was sensitised by subcutaneous injection at the base of tail with a 100 µl dose containing 25 µl PBS, 50 µl (100 µg) endotoxin-free ovalbumin (Worthington Biochemical Corporation, NJ, USA), and 25 µl of adjuvant (I-alum: 2.0 mg; Co₃O₄NP: 25 µg). PBS (100 µl) and Co₃O₄NP (25 µg in PBS with 5% mouse serum) were used for negative controls. Then, 50 µg of OVA in 100 µl of PBS was injected into the peritoneum as a challenge. At 7 days after the second sensitisation, blood was collected from the tail vein. At 7 days after a challenge, the blood was collected by cardiac puncture and peritoneal lavage was performed three times using a 2 ml of 0.9% saline. The first lavage fluid was kept for immunoglobulins and cytokines ELISA, whilst cells from three lavages were pooled for counting cells. The site of injection was also fixed with 10% neutral buffered formalin for histological analysis. *2.5. Immunoglobulin ELISA.* Blood samples collected 1 wk after immunisation and 1 wk after challenge were analysed for the presence of OVA-specific immunoglobulins of IgA, IgE, IgG1, IgG2c (formerly known as IgG2a^{b}) and IgM subclasses. Serum and peritoneal lavage samples were diluted as appropriate in PBS with 1% BSA. Biotin-conjugated secondary antibodies were diluted as follows: anti-IgA - 1:1000; anti-IgG1 - 1:8000; anti-IgG2c - 1:1000; anti-IgM - 1:1000; anti-IgE - 1:250. Before measuring IgE, serum was incubated for an hour with protein-G coupled beads to remove IgG [17;18].

### Cytokine ELISA.

To evaluate the cytokine profile, Th1 type cytokine (IFN-γ), Th2 type cytokines (IL-10 and IL-13), and macrophage-derived pro-inflammatory cytokine (IL-1β) were tested in the peritoneal lavage. All kits were purchased from R&D Systems (Abingdon, UK) and cytokines were measured according to the manufacturer's instruction.

### Differential cell counts of peritoneal lavage.

Total cell number in lavage fluid was assessed by use of a cell nucleus counter (Chemometec, Surrey, UK). Cells were cytospined onto glass slides (Thermo Scientific, Cramlington, UK), fixed with methanol and stained with Diff-Quik (Raymond Lamb, Eastbourne, UK). Around 300 - 500 cells per slide were counted under a light microscope according their morphology.

### Lymphocyte proliferation assay.

Spleens were removed from mice and single cell suspensions made by passing through 40 µm filters. Cells were layered onto lympholyte-M™ (Fisher Scientific, Loughborough, UK) density gradient separation medium and spun at 2000 × g for 15 minutes to remove dead cells, erythrocytes and granulocytes. The mononuclear cell layer at the interface was removed washed and resuspended in tissue culture medium (RPMI 1640 supplemented with 10% FCS, 100 U/ml penicillin, 100 µg/ml streptomycin, 2 mM L-glutamine and 50 µM 2-ME; Sigma, UK). Viable cells were counted and triplicate cultures containing 4 × 10⁵ cells in 200 µl containing no or 10 µM endotoxin-free ovalbumin were plated in 96-well plates. Cultures were incubated for 48 hrs and 0.5 µCi³-H-thymidine was added to each well overnight. Cultures were harvested and incorporated thymidine counted in a betaplate scintillation counter (Wallac UK, Milton Keynes, UK). Supernatants from parallel non-labelled cultures were harvested at 72 hrs for cytokine analysis.

### Histology.

Paraffin embedded formalin fixed tissues from the injection site were cut into 3 µm sections and stained with haematoxylin and eosin.

### Statistical analysis.

Data were analysed with GraphPad Prism Software (Version 5, GraphPad Software Inc., La Jolla, CA, USA). To compare treatment groups, one-way analysis of variance with post hoc Tukey's pairwise comparisons was applied. P < 0.05 was considered to be statistically significant.

### RESULTS

### Physicochemical properties of particles.

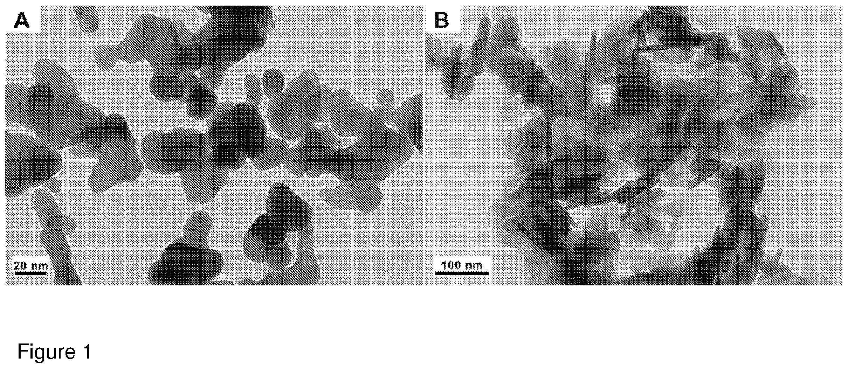

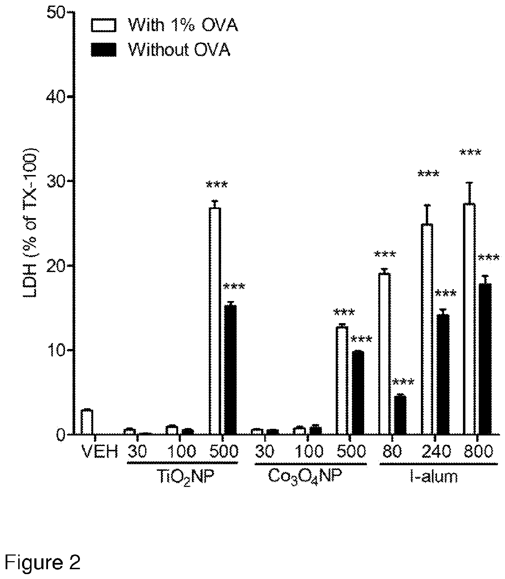

Co₃O₄NP showed spherical shapes and the primary size was 18.4 ± 5.0 nm. I-alum showed more heterogeneous shapes and the average size was 88.7 ± 32.4 nm for length and 28.9 ± 19.7 nm for width (Figure 1). Endotoxin levels of both particles were below the detection limit (0.1 EU/ml). Co₃O₄NP showed large agglomerates without a dispersion medium but small agglomerates with ovalbumin as a dispersant (Table 1). I-alum showed small agglomerates even without dispersion medium. Most I-alum was dissolved within 3 days in the acidic artificial lysosomal fluid (pH 5.5) whilst minimally dissolved in the artificial interstitial fluid (pH 7.4) (data not shown). Co₃O₄NP showed minimal dissolution in both conditions (data not shown). *3.2. In vitro cytotoxicity.* I-alum was by far the most toxic of the NPs tested on mouse macrophages (Figure 2). When particles were dispersed with OVA, particles were more toxic.

**Table 1. Hydrodynamic size and polydispersity of particles 24 h after dispersion (mean ± SD).**

| Dispersion | Co₃O₄NP | | Imject Alum | |
|---|---|---|---|---|
| | Hydrodynamic size | Polydispersity | Hydrodynamic size | Polydispersity |
| Saline | 943.0 ± 251.1 | 0.15 ± 0.01 | 218.9 ± 119.2 | 0.23 ± 0.06 |
| Ovalbumin 0.1% | 90.3 ±17.9 | 0.16 ± 0.02 | 241.2 ± 62.1 | 0.29 ± 0.03 |
| Ovalbumin 1% | 99.8 ±32.6 | 0.20 ± 0.02 | 126.2 ± 36.9 | 0.19 ± 0.05 |

### In vivo immunisation

### Ovalbumin-specific immunoglobulin levels in the serum after sensitisation.

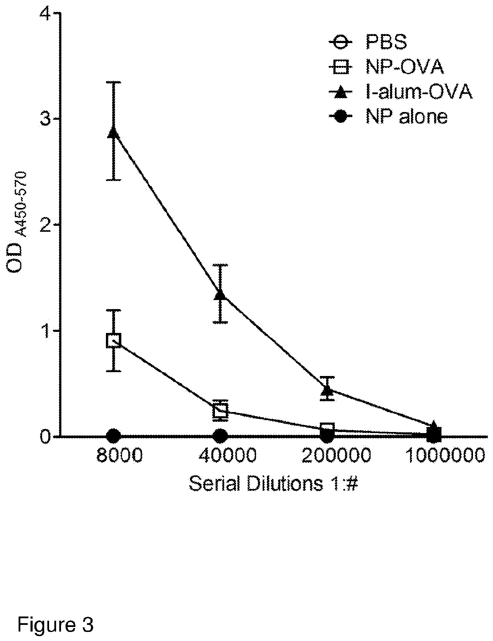

IgG1 was the only immunoglobulin subclass measurable at this stage. Figure 3 shows that both I-alum-OVA and NP-OVA induced IgG1 production although I-alum-OVA was more potent than NP-OVA. Levels of OVA-specific immunoglobulins induced by NP-alone were similar to the vehicle control (PBS).

### Ovalbumin-specific immunoglobulin levels in the serum after challenge.

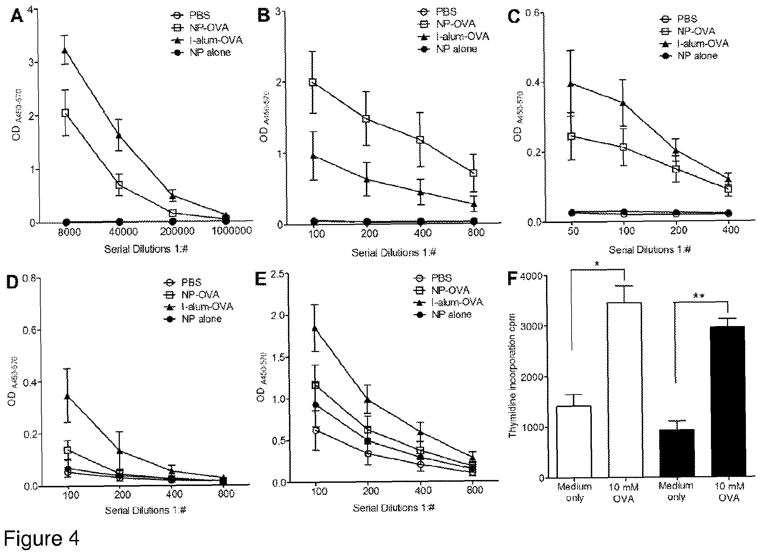

Immunoglobulin levels after challenge demonstrated a wider array of responses. Both NP-OVA and I-alum-OVA produced increases in the IgG1 response although again I-alum-OVA showed higher responses than NP-OVA (Figure 4A). The IgG1 levels in both NP-OVA and I-alum-OVA groups showed a marked increase when compared to those of post-sensitisation. In addition, NP-OVA increased in IgG2c levels (Figure 4B). I-alum-OVA induced higher IgE, IgM, and IgA levels than NP-OVA (Figures 4C - 4E).

### Splenocyte proliferation after challenge.

Spleen mononuclear cells were challenged in vitro with OVA 7 days after challenge. Figure 4F shows that splenocytes from both I-alum and NP-OVA challenged mice proliferated.

### Ovalbumin-specific immunoglobulin levels in the peritoneal lavage after challenge.

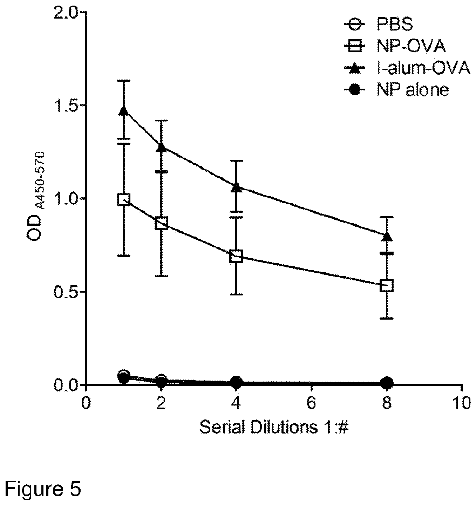

Both NP-OVA and I-alum-OVA showed increases in the IgG1 levels (Figure 5). I-alum-OVA showed higher IgG1 levels compared to NP-OVA. Other immunoglobulins (IgG2c, IgE, IgA, and IgM) were detected only at very low concentrations similar with PBS group (data not shown).

### Cytokine expression in the peritoneal lavage fluid.

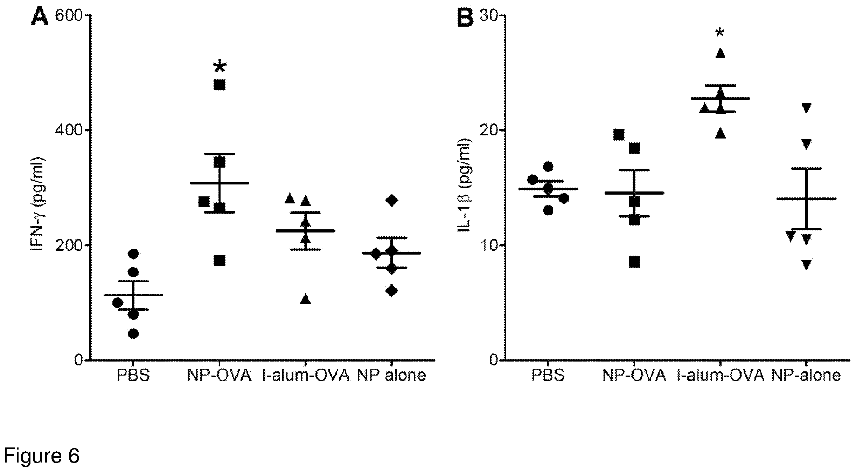

The peritoneal lavage fluid collected from the mice was also used to measure cytokine expression. Levels of IFN-γ were significantly increased by the NP-OVA whilst other groups showed no significant responses (Figure 6A). Levels of IL-1β were significantly increased by I-alum-OVA only (Figure 6B). IL-10 and IL-13 showed no significant changes compared to controls (data not shown).

### Differential cell counts in peritoneal Lavage.

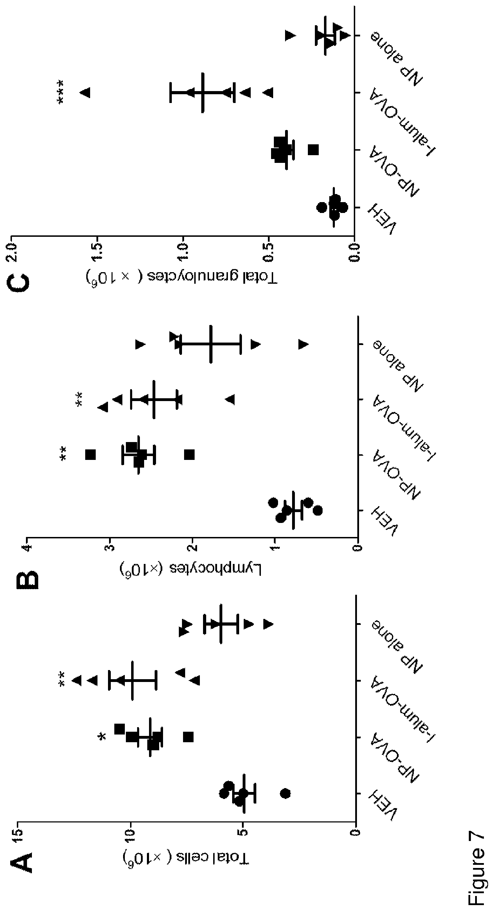

Number of total cells and lymphocytes was significantly increased by both NP-OVA and I-alum-OVA whilst NP-alone was comparable to vehicle control (Figures 7A and 7B). Number of total granulocytes was significantly increased only by I-alum-OVA (Figure 7C).

### Inflammation at the injection site.

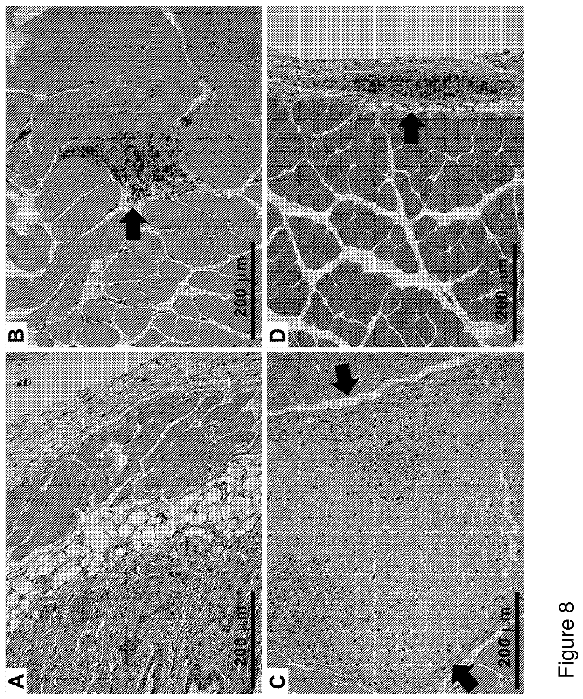

Histology samples taken from the injection site at 7 days after challenge provided sharply contrasting results between the two particles tested. The I-alum-OVA treatment group showed marked inflammation and necrosis with no obvious particle deposition whilst the NP-OVA and NP-alone group showed minimal inflammation with surrounding particle deposition (Figure 8).

### Example 2

### Aims

- Determine the toxicity of Cobalt Oxide nanoparticles and I-Alum to PBMC derived dendritic cells using the Lactate Dehydrogenase Cytotoxicity Assay
- Determine the dose required to induce a good cytokine response of IL-1β, IL-10 and IL-12 without inducing toxicity in the cells
- Treat Bovine PBMC derived Dendritic cells at the optimum concentrations of NP/I-Alum ±Ag85. Ag85 is highly conserved and the protein is identical in the organism which causes human tuberculosis (MTb) and the related organism (MB) that causes TB in cattle. This will allow us to identify the profile of immunomodulatory cytokines produced by the cells by ELISA and assess cell surface maturation markers and MHC Class 11 expression by Flow Cytometric analysis

### Results

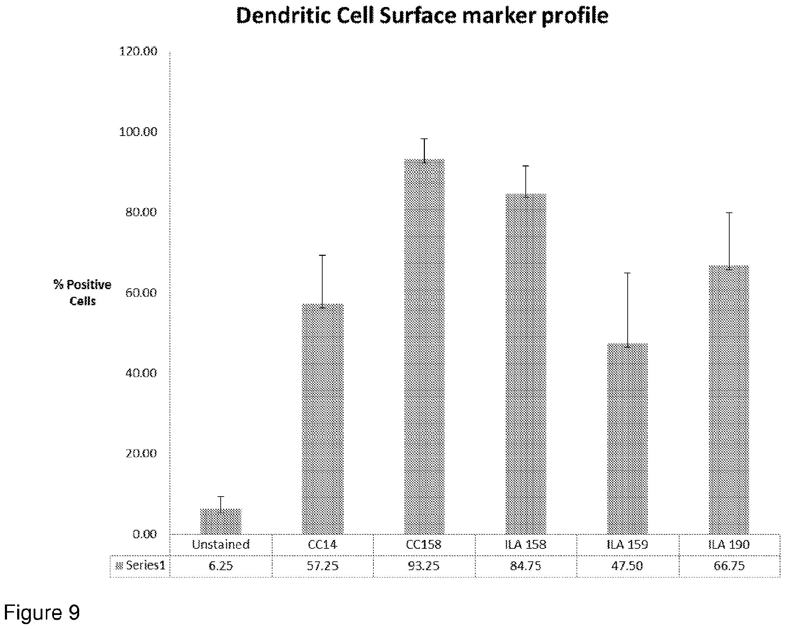

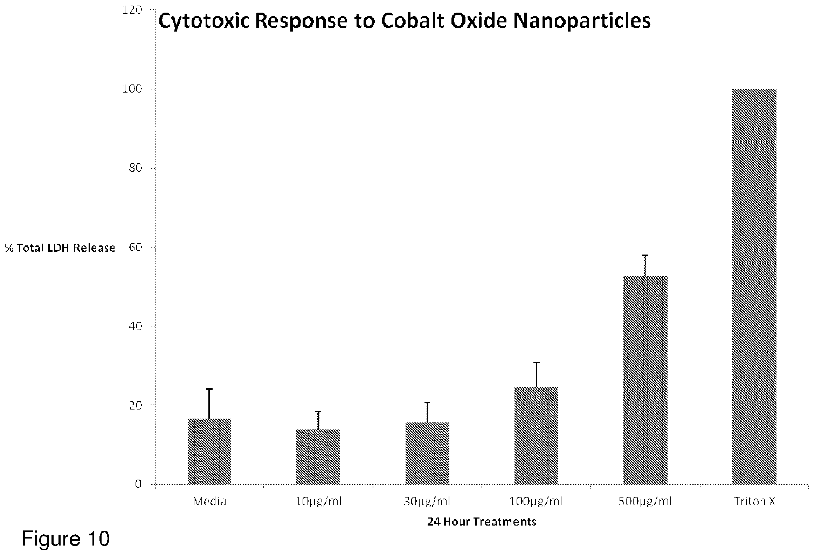

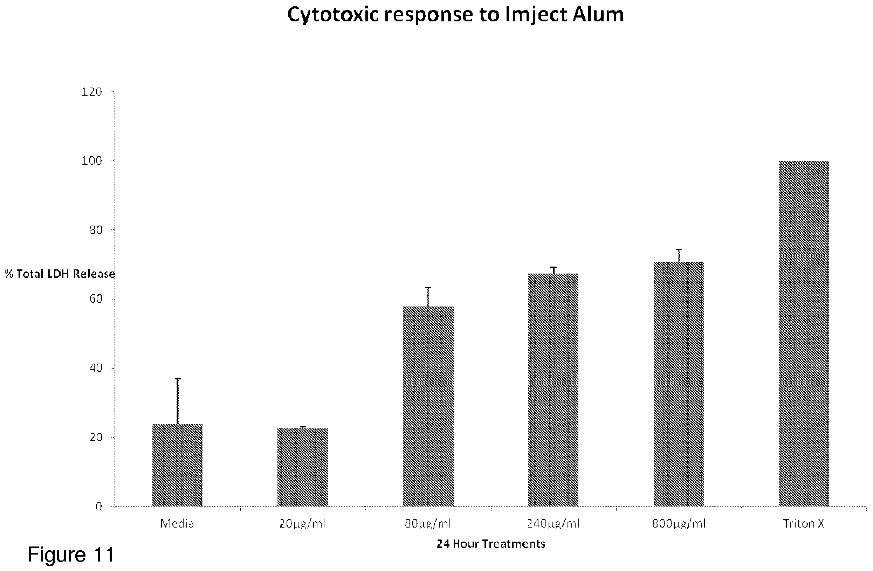

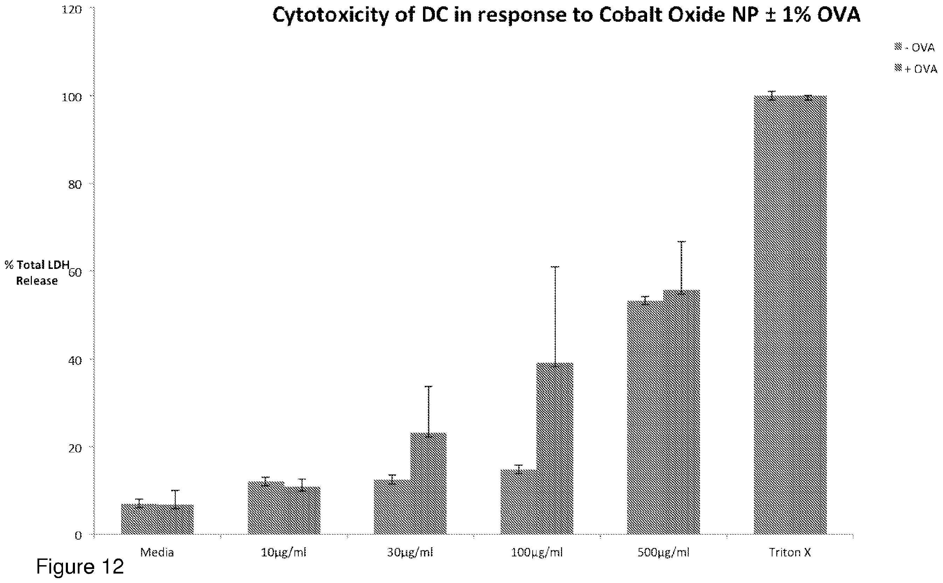

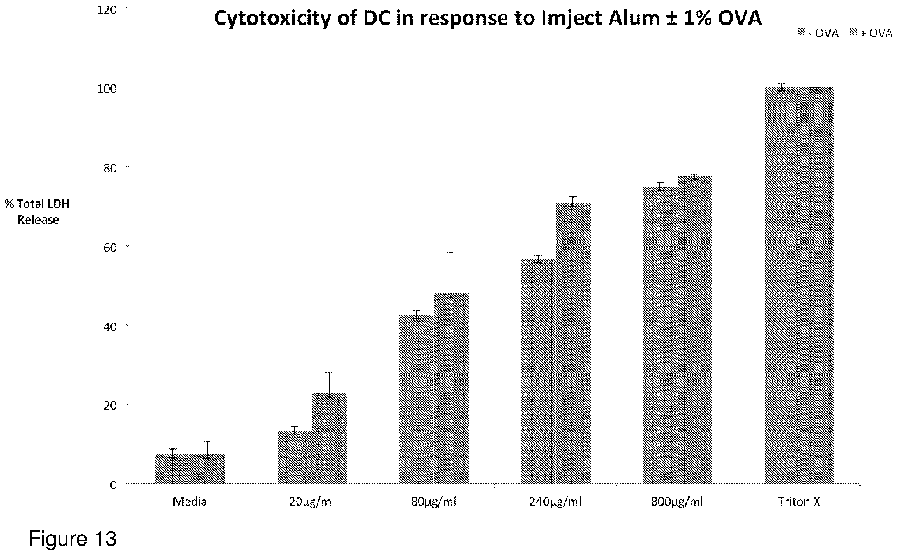

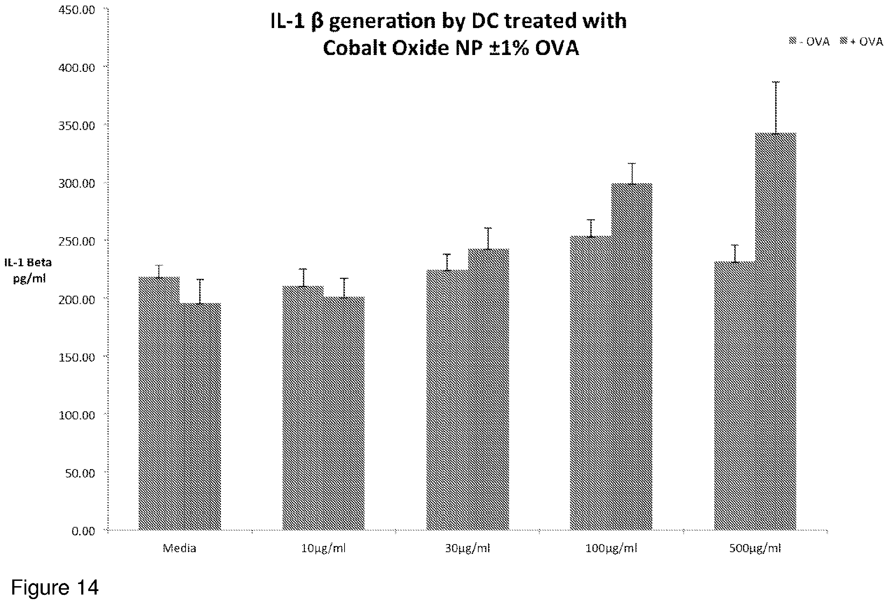

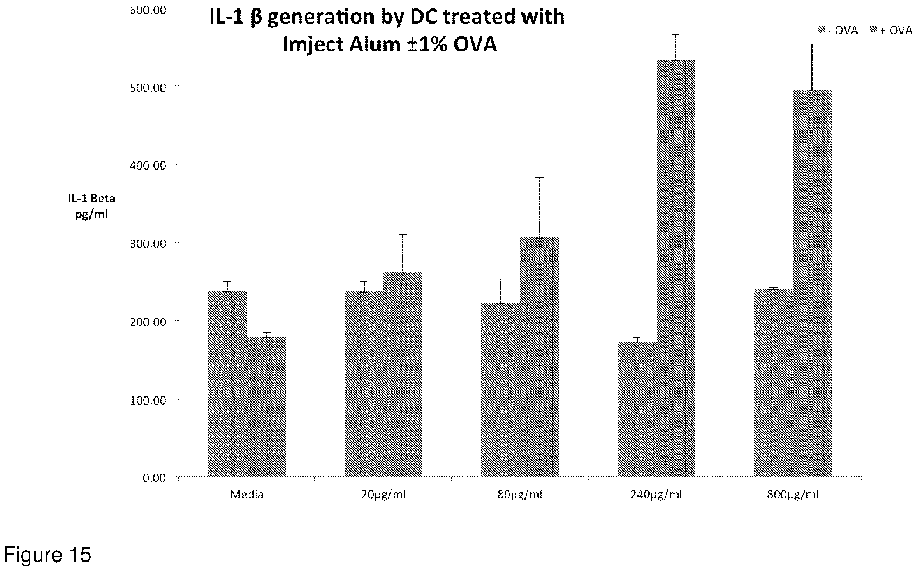

See Figures 9-15 and Tables 2 and 3 (a-f) below.

**Table 2: Hydrodynamic size and Polydispersity**

| | PBS | | | |
|---|---|---|---|---|
| | Media 2% FBS | | Media 5% FBS | |
| | Mean diameter nm | Polydispersity | Mean diameter nm | Polydispersity |
| Imject-Alum | | | | |
| Co₃O₄ NP | 1780 | 0.081 | 567 | 0.076 |

| | MQ H20 | | | |
|---|---|---|---|---|
| | Media 2% FBS | | Media 5% FBS | |
| | Mean diameter nm | Polydispersity | Mean diameter nm | Polydispersity |
| Imject-Alum | 337 | 0.138 | 193 | 0.28 |
| Co₃O₄NP | 66 | 0.187 | 136 | 0.005 |

**Table 3 (a-f): Cytokine response to Cobalt Oxide Nanoparticles and Imject-Alum (n=4).**

| **3A** | | |
|---|---|---|
| **NP** | **Average** | **SEM** |
| Media | 17.30 | 11.74 |
| 10µg/ml | 36.42 | 34.80 |
| 30µg/ml | 8.64 | 7.26 |
| 100µg/ml | 140.71 | 121.57 |
| 500µg/ml | 10.50 | 6.99 |
| | | |

| **3B** | | |
|---|---|---|
| **NP** | **Average** | **SEM** |
| Media | 0.19 | 0.05 |
| 10µg/ml | 0.34 | 0.18 |
| 30µg/ml | 0.27 | 0.23 |
| 100µg/ml | 0.56 | 0.42 |
| 500µg/ml | 0.26 | 0.12 |
| | | |

| **3C** | | |
|---|---|---|
| **NP** | **Average** | **SEM** |
| Media | 0.14 | 0.12 |
| 10µg/ml | 0.09 | 0.08 |
| 30µg/ml | 7.8 | 3.49 |
| 100µg/ml | 0.06 | 0.05 |
| 500µg/ml | 1.15 | 0.27 |
| | | |

| **3D** | | |
|---|---|---|
| **I-ALUM** | **Average** | **SEM** |
| Media | 14.68 | 12.27 |
| 20µg/ml | 19.75 | 9.36 |
| 80µg/ml | 22.30 | 9.43 |
| 240µg/ml | 52.76 | 20.05 |
| 800µg/ml | 54.61 | 12.2 |
| | | |

| **3E** | | |
|---|---|---|
| **I-ALUM** | **Average** | **SEM** |
| Media | 0.44 | 0.23 |
| 20µg/ml | 0.08 | 0.05 |
| 80µg/ml | 0.21 | 0.19 |
| 240µg/ml | 0.19 | 0.15 |
| 800µg/ml | 0.17 | 0.12 |

| **3F** | | |
|---|---|---|
| **I-ALUM** | **Average** | **SEM** |
| Media | 0.14 | 0.14 |
| 20µg/ml | 0.21 | 0.18 |
| 80µg/ml | 10.85 | 10.61 |
| 240µg/ml | 0.2 | 0.12 |
| 800µg/ml | 0.75 | 0.69 |

### Summary

- Successfully determined the cell surface marker profile for resting DCS. This was then compared to DCS treated with Cobalt Oxide nanoparticles and Imject Alum ± Ag85 to address changes in activation status and MHC Class 11 expression
- Using the Lactate Dehydrogenase Cytotoxity Assay we determined the levels of toxicity against increasing doses of Cobalt Oxide nanoparticles and Imject Alum. The concentration of each will be 50µg/ml
- Looked at the profile of immunomodulatory cytokines produced by the cells by ELISA. Increased levels of IL-1β being produced in response to greater doses of Imject Alum.
- There is no difference in the levels of cellular toxicity in Dendritic cells treated with Cobalt Oxide NP's and I - Alum (± 1% OVA)
- The levels of IL-1β in the supernatants from Dendritic cells treated with Cobalt Oxide NP's and I - Alum are comparable for both the Cobalt Oxide and Imject Alum treated DCs
- The addition of 1% OVA leads to increased levels of IL-1 Beta being produced with Imject Alum + 1% OVA showing higher levels compared to Cobalt Oxide NP + 1% OVA.

### DISCUSSION

The safety of adjuvants to be used on human is a key issue in their development. Recent studies have shown that the licensed adjuvant activates human monocytes and macrophages[20], suggesting that what would be deemed as toxicity is potentially a part of the mechanism by which alum adjuvant works. In this study, we hypothesised that Co₃O₄NP may produce a balanced Th1 and Th2 response with less toxicity than alum, suggesting that they may be a safer option.

Co₃O₄NP showed relatively homogenous spherical shape of particles. Interestingly, I-alum was more heterogeneous and less than 100 nm in both dimensions. The heterogeneous shape might be due to the composition of I-alum comprising a mixture of aluminium hydroxide and magnesium hydroxide. Therefore, both Co₃O₄NP and I-alum were within the definition of nanoparticles and classified as metal oxide NPs[21;22]. As previous studies have shown that size matters in adjuvanticity, optimal dispersion of NPs might be beneficial[12]. OVA acted as an excellent dispersant for both particles. Co₃O₄NP showed small agglomerates when dispersed with OVA which forms a protein corona providing repulsive force between particles[23]. In contrast to Co₃O₄NP, I-alum was relatively well-dispersed in saline which might be because of the stabilisers added during manufacturing.

Adjuvants are believed to work by activating antigen presenting cells but there is little information on their toxicity for such cells. Using a murine macrophage cell line, the LDH cytotoxicity study showed that I-alum by itself was approximately 6 times more toxic than an equivalent amount of Co₃O₄NP. Dispersal in OVA showed increased toxicity of both Co₃O₄NP and alum which might be due to the cytotoxicity of OVA itself or to facilitating recognition and phagocytosis of particles by forming a protein corona.

The post-sensitisation data showed that both I-alum-OVA and NP-OVA increased serum OVA-specific IgG1 levels (which is marker for Th2 response[3]) whilst other immunoglobulins were not increased. However, the potential for IgG1 production by I-alum-OVA was about three times higher than that of NP-OVA. The post-challenge data showed that a T cell response was generated, confirmed by proliferation of spleen cells cultured with OVA *in vitro.* Further boosted immunoglobulin responses were also seen in mice immunized with either adjuvant. The presence of antigen specific class-switched immunoglobulins (IgG and IgE) in serum indicate that both Th1 and Th2 OVA-specific CD4+ T lymphocytes have been activated by OVA after sensitisation with the antigen using Co₃O₄NP as adjuvant. In serum I-alum-OVA produced mainly Th2 type (IgG1) and allergic antibody responses (IgE) whilst NP-OVA produced a more balanced Th1 (IgG2c) and Th2 (IgG1) type response with much less IgE. This suggests that Co₃O₄NP as an adjuvant maybe more likely to provide robust 'all-round' immunity and less likely to induce unwanted allergic/side effects. This was further supported by the post-challenge peritoneal lavage fluid analysis. Th2 dependent IgG1 levels were significantly increased by NP-OVA and I-alum-OVA with the same pattern as seen in serum. Peritoneal lavage IFN-γ a Th1 type cytokine[24], was increased only in the NP-OVA immunised group. The differential cell counts of the peritoneal lavage fluid showed that both NP-OVA and alum-OVA induced lymphocytic inflammation which is important for adjuvanticity[5] but NP-OVA induced far less granulocytic inflammation. This granulocytic inflammation by 1-alum-OVA was consistent with the increased IL-1β in the peritoneal lavage which is one of the macrophage derived pro-inflammatory cytokines. The pattern of response induced by I-alum-OVA is consistent with that seen in other studies[25;26].

At 7 days after challenge at the doses used, I-alum induced prolonged massive inflammation and necrosis in the subcutaneous and muscular layer of the injected skin whilst Co₃O₄NP showed mild inflammation. The greater inflammation induced by I-alum compared to Co₃O₄NP was consistent with increased peritoneal IL-1β secretion and number of granulocytes recruited to the peritoneal cavity by I-alum indicating inflammatory responses at both sensitisation and challenge sites. In addition the I-alum induced more allergic anti-OVA IgE serum antibodies. This provides a more rounded result with Co₃O₄NP than a previous report where TiO₂NP showed a higher IgE response than Al(OH)₃, though in that case Al(OH)₃ produced the higher granulocyte recruitment concurring with our data[27]. In addition, Co₃O₄NP showed a more balanced Th1 and Th2 response even with 80 times less mass administered than I-alum. The absence of cell death and inflammation at the injection site of NP-OVA group indicates that it will cause less local pain. In addition from a veterinary point of view in food animals, less cell death and inflammation at the injection site means less and hide meat spoilage and may have improved economic benefits.

Within 3 days of incubation, most of the I-alum was dissolved in the acidic (pH 5.5) solution which is the same pH of the lysosomal fluid of alveolar macrophages[28] whilst there was minimal dissolution in the basic (pH 7.4) solution. However, Co₃O₄NP showed minimal dissolution in both conditions. Compositional ions released in the acidic lysosomal condition would be likely to play a role in cell death and inflammation for high-solubility NPs such as CuONP[29] and ZnONP[30]. Therefore, Al³⁺ dissolved in the lysosomes of phagocytic cells might contribute to cell death and further inflammation by Trojan-Horse type mechanism[31]. Co₃O₄NP had not changed in solubility and this was consistent with persistence at the injection site. This suggests that the Co₃O₄NP is very insoluble and stable. There is a concern regarding the solubility of Co₃O₄NP, as cobalt ions are being released very slowly into cells and their long-term effect is unknown[19]. Cobalt poisoning is not common but it can occur. There are reports of hip replacements (which contain cobalt and chromium) that corrode over time and release metal ions into the body causing severe illness[32]. This seems an unlikely side-effect here for Co₃O₄NP due to their small dose and volume of injection and insoluble nature. In our previous study, deposition of Co₃O₄NP inside of lung causes delayed-type hypersensitivity and pulmonary alveolar lipoproteinosis[19]. However considering the lung has much more active immune response than the skin and muscle, the subcutaneous side effects might be more limited and indeed only mild inflammation was produced in the injection site at 5 weeks after two injections. However if multiple repeated vaccinations were to be administered, build-up of Co₃O₄NP and release of cobalt ions may cause health problems. Accumulation of the Co₃O₄NP within the subcutaneous and muscular layers indicates that Co₃O₄NP may provide a continuous source of desorbed antigen which is one of the modes of actions proposed for alum adjuvant[33].

The comparison between I-alum and Co₃O₄NP is suitable for measuring a response in mice. Imject Alum is not licensed for use in human and has a different physicochemistry to clinically approved products[33]. To be able to fully evaluate Co₃O₄NP in long-term study as a prospective human adjuvant further studies are required but we believe that Co₃O₄NP shows great promise in inducing both Th1 and Th2 mediated responses.

### CONCLUSION

In conclusion, Co₃O₄NP stimulated less allergic antibody production and *in vivo* inflammation (at both sensitisation and challenge sites) than a standard dose of the alum-containing adjuvant Imject alum. Together with the evidence that they also produced lower *in vitro* toxicity whilst stimulating both Th1 and Th2 *in vivo* antibody responses, this indicates that Co₃O₄NP would be suitable for use as a vaccine adjuvant.

### References

1. Gupta RK, Siber GR. Adjuvants for human vaccines--current status, problems and future prospects. Vaccine. 13, 1263-1276 (1995).
2. Schijns VE, Brewer JM. New views on immunopotentiators in modern vaccines. Expert.Rev. Vaccines. 7, 877-879 (2008).
3. Cox JC, Coulter AR. Adjuvants--a classification and review of their modes of action. Vaccine. 15, 248-256 (1997).
4. Lambrecht BN, Kool M, Willart MA, Hammad H. Mechanism of action of clinically approved adjuvants. Curr.Opin.Immunol. 21, 23-29 (2009).
5. MacLeod MK, McKee AS, David A, et al. Vaccine adjuvants aluminum and monophosphoryl lipid A provide distinct signals to generate protective cytotoxic memory CD8 T cells. Proc.Natl.Acad.Sci.U.S.A. 108, 7914-7919 (2011).
6. Leucuta SE. Nanotechnology for delivery of drugs and biomedical applications. Curr.Clin.Pharmacol. 5, 257-280 (2010).
7. Lundqvist M, Stigler J, Elia G, Lynch I, Cedervall T, Dawson KA. Nanoparticle size and surface properties determine the protein corona with possible implications for biological impacts. Proc.Natl.Acad.Sci.U.S.A. 105, 14265-14270 (2008).
8. Hallaj-Nezhadi S, Lotfipour F, Dass C. Nanoparticle-mediated interleukin-12 cancer gene therapy. J.Pharm.Pharm.Sci. 13, 472-485 (2010).
9. Brewer JM. (How) do aluminium adjuvants work? Immunol.Lett. 102, 10-15 (2006).
10. Fahmy TM, Demento SL, Caplan MJ, Mellman I, Saltzman WM. Design opportunities for actively targeted nanoparticle vaccines. Nanomedicine.(Lond). 3, 343-355 (2008).
11. Mamo T, Moseman EA, Kolishetti N, et al. Emerging nanotechnology approaches for HIV/AIDS treatment and prevention. Nanomedicine.(Lond). 5, 269-285 (2010).
12. Li X, Sloat BR, Yanasarn N, Cui Z. Relationship between the size of nanoparticles and their adjuvant activity: data from a study with an improved experimental design. Eur.J.Pharm.Biopharm. 78, 107-116 (2011).
13. Tiwari S, Agrawal GP, Vyas SP. Molecular basis of the mucosal immune system: from fundamental concepts to advances in liposome-based vaccines. Nanomedicine.(Lond). 5, 1617-1640 (2010).
14. Inoue K, Koike E, Yanagisawa R, Hirano S, Nishikawa M, Takano H. Effects of multi-walled carbon nanotubes on a murine allergic airway inflammation model. Toxicol.Appl.Pharmacol. 237, 306-316 (2009).
15. Dong CC, Yin XJ, Ma JY, et al. Exposure of brown Norway rats to diesel exhaust particles prior to ovalbumin (OVA) sensitization elicits IgE adjuvant activity but attenuates OVA-induced airway inflammation. Toxicol.Sci. 88, 150-160 (2005).
16. de Haar C, Hassing I, Bol M, Bleumink R, Pieters R. Ultrafine carbon black particles cause early airway inflammation and have adjuvant activity in a mouse allergic airway disease model. Toxicol.Sci. 87, 409-418 (2005).
17. Leech MD, Benson RA, De VA, Fitch PM, Howie SE. Resolution of Der p1-induced allergic airway inflammation is dependent on CD4+CD25+Foxp3+ regulatory cells. J.Immunol. 179, 7050-7058 (2007).
18. Wilson MS, Taylor MD, Balic A, Finney CA, Lamb JR, Maizels RM. Suppression of allergic airway inflammation by helminth-induced regulatory T cells. J.Exp.Med. 202, 1199-1212 (2005).
19. Cho WS, Duffin R, Bradley M, et al. NiO and Co3O4 Nanoparticles Induce Lung DTH-Like Responses and Alveolar Lipoproteinosis. Eur Respir J erj00471-2011; published ahead of print doi:10.1183/09031936.00047111 (2011).
20. De Gregorio E, Tritto E, Rappuoli R. Alum adjuvanticity: unraveling a century old mystery. Eur.J.Immunol. 38, 2068-2071 (2008).
21. Donaldson K, Stone V, Tran CL, Kreyling W, Borm PJ. Nanotoxicology. Occup.Environ.Med. 61, 727-728 (2004).
22. Stone V, Donaldson K. Nanotoxicology: signs of stress. Nat.Nanotechnol. 1, 23-24 (2006).
23. Bihari P, Vippola M, Schultes S, et al. Optimized dispersion of nanoparticles for biological in vitro and in vivo studies. Part Fibre.Toxicol. 5, 14 (2008).
24. Meyts I, Hellings PW, Hens G, et al. IL-12 contributes to allergen-induced airway inflammation in experimental asthma. J.Immunol. 177, 6460-6470 (2006,).
25. McKee AS, MacLeod M, White J, Crawford F, Kappler JW, Marrack P. Gr1+IL-4-producing innate cells are induced in response to Th2 stimuli and suppress Th1-dependent antibody responses. Int.Immunol. 20, 659-669 (2008).
26. Brewer JM, Conacher M, Hunter CA, Mohrs M, Brombacher F, Alexander J. Aluminium hydroxide adjuvant initiates strong antigen-specific Th2 responses in the absence of IL-4- or IL-13-mediated signaling. J.Immunol. 163, 6448-6454 (1999).
27. Larsen ST, Roursgaard M, Jensen KA, Nielsen GD. Nano titanium dioxide particles promote allergic sensitization and lung inflammation in mice. Basic Clin.Pharmacol.Toxicol. 106, 114-117 (2010).
28. Nyberg K, Johansson U, Johansson A, Camner P. Phagolysosomal pH in alveolar macrophages. Environ.Health Perspect. 97, 149-152 (1992).
29. Studer AM, Limbach LK, Van Duc L, et al. Nanoparticle cytotoxicity depends on intracellular solubility: comparison of stabilized copper metal and degradable copper oxide nanoparticles. Toxicol.Lett. 197, 169-174 (2010).
30. Muller KH, Kulkarni J, Motskin M, et al. pH-dependent toxicity of high aspect ratio ZnO nanowires in macrophages due to intracellular dissolution. ACS Nano. 4, 6767-6779 (2010).
31. Ganrot PO. Metabolism and possible health effects of aluminum. Environ.Health Perspect. 65, 363-441 (1986).
32. Witzleb WC, Ziegler J, Krummenauer F, Neumeister V, Guenther KP. Exposure to chromium, cobalt and molybdenum from metal-on-metal total hip replacement and hip resurfacing arthroplasty. Acta Orthop. 77, 697-705 (2006).
33. Exley C, Siesjo P, Eriksson H. The immunobiology of aluminium adjuvants: how do they really work? Trends Immunol. 31, 103-109 (2010).

## Claims

1. An adjuvant comprising cobalt nanoparticles and/or cobalt oxide nanoparticles.

2. The adjuvant of claim 1, wherein the adjuvant does not comprise alum.

3. The adjuvant of claim 1 or 2, wherein the cobalt oxide nanoparticles comprise cobalt (II,III) oxide (Co₃O₄) nanoparticles.

4. The adjuvant of any preceding claim, wherein the adjuvant comprises or further comprises cobaltous oxide (Cobalt (II) oxide: CoO) nanoparticles and/or cobaltic oxide (Cobalt (III) oxide: Co₂O₃) nanoparticles.

5. The adjuvant of any preceding claims, wherein the nanoparticles are between 1 nm and 100 nm in diameter.

6. The adjuvant of any preceding claim, wherein the cobalt nanoparticles have an average diameter of 18.4 ± 5.0 nm.

7. Inorganic cobalt nanoparticles and/or cobalt oxide nanoparticles for use as adjuvants.

8. The inorganic cobalt nanoparticles and/or cobalt oxide nanoparticles of claim 7 for use of claim 7, wherein the cobalt nanoparticles and/or cobalt oxide nanoparticles comprise Co₃O₄ nanoparticles.

9. An adjuvant composition comprising the adjuvant of any one of claims 1-6, a pharmaceutically or physiologically acceptable excipient, solvent, carrier or diluent.

10. The adjuvant composition of claim 9, wherein the composition comprises one or more additional adjuvant components.

11. A vaccine comprising an adjuvant of any one of claims 1-6.

12. An adjuvant according to any one of claims 1-6 or vaccine according to claim 11, for use in (i) enhancing immune responses in human or animal subjects, (ii) raising immune responses and/or (iii) enhancing, modulating or augmenting immune responses, in human or animal subjects.

## Patentansprüche

1. Adjuvans, beinhaltend Cobalt-Nanopartikel und/oder Cobaltoxid-Nanopartikel.

2. Adjuvans nach Anspruch 1, bei welchem das Adjuvans kein Alaun beinhaltet.

3. Adjuvans nach Anspruch 1 oder 2, bei welchem die Cobaltoxid-Nanopartikel Cobalt(II,III)-oxid (Co₃O₄)-Nanopartikel beinhalten.

4. Adjuvans nach einem der vorhergehenden Ansprüche, bei welchem das Adjuvans Cobaltoxid (Cobalt(II)-oxid: CoO)-Nanopartikel und/oder Cobaltoxid (Cobalt(III)-oxid: Co₂O₃)-Nanopartikel beinhaltet oder zudem beinhaltet.

5. Adjuvans nach einem der vorhergehenden Ansprüche, bei welchem die Nanopartikel einen Durchmesser zwischen 1 nm und 100 nm aufweisen.

6. Adjuvans nach einem der vorhergehenden Ansprüche, bei welchem die Nanopartikel einen mittleren Durchmesser von 18.4 ± 5.0 nm aufweisen.

7. Anorganische Cobalt-Nanopartikel und/oder Cobaltoxid-Nanopartikel zum Gebrauch als Adjuvanzien.

8. Anorganische Cobalt-Nanopartikel und/oder Cobaltoxid-Nanopartikel nach Anspruch 7 zum Gebrauch nach Anspruch 7, bei welchen die Cobalt-Nanopartikel und/oder Cobaltoxid-Nanopartikel Co₃O₄-Nanopartikel beinhalten.

9. Adjuvans-Zusammensetzung, beinhaltend das Adjuvans nach einem der Ansprüche 1 bis 6, ein(en) pharmazeutisch oder physiologisch akzeptables(en) Hilfsstoff, Lösemittel, Trägerstoff oder Verdünnungsmittel.

10. Adjuvans-Zusammensetzung nach Anspruch 9, bei welcher die Zusammensetzung eine oder mehrere zusätzliche Adjuvans-Komponenten beinhaltet.

11. Impfstoff, beinhaltend ein Adjuvans nach einem der Ansprüche 1 bis 6.

12. Adjuvans nach einem der Ansprüche 1 bis 6 oder Impfstoff nach Anspruch 11, zum Gebrauch zwecks (i) Verbessern der Immunreaktion bei Menschen oder Tieren, (ii) Hervorrufen von Immunreaktionen und/oder (iii) Verbessern, Modulieren oder Steigern von Immunreaktionen bei Menschen oder Tieren.

## Revendications

1. Adjuvant comprenant des nanoparticules de cobalt et/ou des nanoparticules d'oxyde de cobalt.

2. Adjuvant selon la revendication 1, dans lequel l'adjuvant ne comprend pas d'alun.

3. Adjuvant selon la revendication 1 ou 2, dans lequel les nanoparticules d'oxyde de cobalt comprennent des nanoparticules d'oxyde (Co₃O₄) de cobalt (II, III).

4. Adjuvant selon l'une quelconque des revendications précédentes, dans lequel l'adjuvant comprend ou comprend en outre des nanoparticules d'oxyde cobalteux (oxyde de cobalt (II) : CoO) et/ou des nanoparticules d'oxyde cobaltique (oxyde de cobalt (III) : Co₂O₃).

5. Adjuvant selon l'une quelconque des revendications précédentes, dans lequel les nanoparticules ont entre 1 nm et 100 nm de diamètre.

6. Adjuvant selon l'une quelconque des revendications précédentes, dans lequel les nanoparticules de cobalt ont un diamètre moyen de 18,4 ± 5,0 nm.

7. Nanoparticules de cobalt inorganiques et/ou nanoparticules d'oxyde de cobalt inorganiques en vue d'une utilisation en tant qu'adjuvants.

8. Nanoparticules de cobalt inorganiques et/ou nanoparticules d'oxyde de cobalt inorganiques selon la revendication 7 en vue d'une utilisation selon la revendication 7, dans lesquelles les nanoparticules de cobalt et/ou les nanoparticules d'oxyde de cobalt comprennent des nanoparticules de Co₃O₄.

9. Composition d'adjuvant comprenant l'adjuvant selon l'une quelconque des revendications 1-6, un excipient, un solvant, un véhicule ou un diluant pharmaceutiquement ou physiologiquement acceptable.

10. Composition d'adjuvant selon la revendication 9, dans laquelle la composition comprend un ou plusieurs composants d'adjuvant supplémentaires.

11. Vaccin comprenant un adjuvant selon l'une quelconque des revendications 1-6.

12. Adjuvant selon l'une quelconque des revendications 1-6 ou vaccin selon la revendication 11, en vue d'une utilisation dans (i) l'amélioration des réponses immunitaires chez des sujets humains ou animaux, (ii) l'augmentation des réponses immunitaires et/ou (iii) l'amélioration, la modulation ou l'augmentation des réponses immunitaires, chez des sujets humains ou animaux.
